## Europäisches Patentamt

## European Patent Office

## Office européen des brevets

(19)

(11) Veröffentlichungsnummer: **0 111 885**

**A1**

(12) **EUROPÄISCHE PATENTANMELDUNG**

(21) Anmeldenummer: **83112559.6**

(22) Anmeldetag: **14.12.83**

(51) Int. Cl.³: **C 12 M 1/08**
**C 12 P 7/06, C 12 M 1/40**

(30) Priorität: **18.12.82 DE 3247214**

(43) Veröffentlichungstag der Anmeldung:
**27.06.84 Patentblatt 84/26**

(84) Benannte Vertragsstaaten:
**AT BE CH DE FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Keller, Reinhold, Dr.**
**Wiesenweg 5**
**D-6232 Bad Soden am Taunus(DE)**

(72) Erfinder: **Faust, Uwe, Dr.**
**Kelkheimer Strasse 27**
**D-6233 Kelkheim (Taunus)(DE)**

(54) Reaktionen mit immobilisierten Biokatalysatoren.

(57) Enzymatische Reaktionen mit immobilisierten Biokatalysatoren werden vorteilhaft in einem Reaktor nach dem Schlaufenprinzip, insbesondere in einem Airliftreaktor, durchgeführt. Reaktionen unter Gasaufnahme und/oder -abgabe oder pH-Verschiebung lassen sich - diskontinuierlich oder kontinuierlich - gut beherrschen und verlaufen über lange Zeit ohne Aktivitätsabfall des Katalysators.

EP 0 111 885 A1

Croydon Printing Company Ltd.

HOECHST AKTIENGESELLSCHAFT          C111825          HOE 82/F 260

Reaktionen mit immobilisierten Biokatalysatoren

Die Erfindung betrifft ein Verfahren zur Durchführung enzymatischer Reaktionen mit immobilisierten Biokatalysatoren, das dadurch gekennzeichnet ist, daß die Reaktion in einem Reaktor nach dem Schlaufenprinzip erfolgt.

Die Immobilisierung von Biokatalysatoren, insbesondere von Mikroorganismen oder Enzymen, und ihre Anwendung in enzymatischen Reaktionen gewinnt zunehmende Bedeutung (Nachr. Chem. Tech. Lab. 29 (1981) 850). Bei der Anwendung dieser immobilisierten Biokatalysatoren kommt es darauf an, diese mit dem Substrat unter möglichst geringem technischem Aufwand so gut wie möglich miteinander in Berührung zu bringen. Im Gegensatz zu nativen Enzymen, die im Reaktionsmedium gelöst werden, oder unmittelbar im Reaktionsmedium suspendierten Mikroorganismen benötigen immobilisierte Präparate eine möglichst große Relativbewegung zwischen Enzym und Substrat. Die bisher bekannten Anwendungsmethoden immobilisierter Biokatalysatoren weisen jedoch alle gewisse Nachteile auf:

Bei der ansatzweisen Anwendung immobilisierter Enzyme im Tankreaktor ist ein intensives Rühren erforderlich. Nachteilig hierbei ist nicht nur der große Energieaufwand, sondern auch die hohe mechanische Beanspruchung der Enzym- bzw. Mikroorganismenpartikel. Weiterhin ist nur eine diskontinuierliche Verfahrensdurchführung möglich.

Beim kontinuierlichen Tankreaktor wird laufend Substrat zu- und Produkt abgeführt. Die im Reaktor suspendierten Teilchen werden mechanisch am Abgang aus dem Reaktionsgefäß gehindert. Auch bei diesem Verfahren wirkt eine hohe mechanische Belastung auf die Biokatalysatoren ein.

In Fließbettreaktoren wird das Substrat im allgemeinen von unten nach oben durch eine Säule gedrückt. Die in der Säule befindlichen immobilisierten Enzyme oder Mikroorganismen werden dann durch die Strömung des Substrats in der Schwebe gehalten. Hierfür ist erforderlich, daß das spezifische Gewicht der Katalysatorpartikel höher ist als das des Substrats.

Der Packbettreaktor enthält die Katalysatorpartikel als angeschwemmte Schicht, durch die das zu behandelnde Substrat hindurch gedrückt wird. Die mechanischen Eigenschaften der immobilisierten Präparate sollen hierbei so sein, daß ein möglichst geringer Druck für das Durchpumpen der Flüssigphase erforderlich ist.

Nachteilig bei beiden Bettreaktoren ist die geringe Durchmischung von Substrat und immobilisiertem Präparat infolge von Rißbildungen und Ungleichmäßigkeiten im Enzymbett. Außerdem können nur klarfiltrierte Substratlösungen eingesetzt werden, da sonst ein Verstopfen und Verunreinigen des Enzymbettes zu befürchten ist. Diese vorgeschaltete Klarfiltration ist bei technischen Prozessen jedoch mit einem erheblichen Aufwand verbunden. Werden bei der Umsetzung Gase freigesetzt oder eingesetzt, so besteht die Gefahr eines Austrocknens des Enzymbettes. Erfolgt bei der Umsetzung eine pH-Verschiebung, so kann sich innerhalb des Enzymbettes ein pH-Gradient ausbilden, was zu einer Verringerung der Aktivität führt.

Bei der erfindungsgemäßen Umsetzung in einem Schlaufenreaktor treten die genannten Nachteile nicht auf. Dieser Reaktortyp ist also sehr vielseitig für Reaktionen mit immobilisierten Biokatalysatoren einsetzbar.

Chemische Reaktoren mit Schlaufencharakteristik sind seit langem bekannt (H.Blenke, W. Hirner, VDI-Bericht 218 (1974) 549). Auch in der Fermentationstechnik werden verstärkt Schlaufenfermenter eingesetzt (A. Prokorp, J. Votruba, Folin. Microbiol. 21 (1976) 58; U.-Faust, W. Sittig, 27. IUPAC-Congress, Ed. A. Varmavouri, Pergamon Press 1980).

Beim Schlaufenreaktor wird ein zunächst heterogenes Reaktionsgemisch durch gerichteten Antrieb in einem Reaktionsraum in eine longitudinale Strömung versetzt und durch die geschlossene Schlaufenkonstruktion des Reaktionsraums im Kreis geführt. Der Antrieb kann durch Beschleunigung des Reaktionsmediums mittels einer in den Kreislauf geschalteten Pumpe, eines Rührwerks mit Fördercharakter (Propellerrührer) oder durch Einpressen von Gasen, beispielsweise Einblasen komprimierter Luft, erfolgen. Im letzteren Fall spricht man von "Airliftschlaufenreaktoren". Bei diesen Airliftschlaufenreaktoren wird der Antrieb durch die Dichtedifferenz zwischen einem begasten und einem unbegasten Teil des Reaktors (Aufstiegs- und Abstiegsteil) erreicht, wobei der Impulsübertrag gleichmäßig über das gesamte Medium verteilt erfolgt. Dadurch erreicht man eine sehr gleichmäßige und schonende Mischung.

Der Airliftschlaufenreaktor ist vor allem dort interessant, wo die Reaktion ein gasförmiges Substrat wie Sauerstoff, Kohlendioxid oder Methan erfordert, da gleichzeitig mit der Antriebsleistung auch der Gastransport von der Gasphase in das Medium erfolgt. Dasselbe gilt für Prozesse, bei denen eine Gasabspaltung erfolgt und demzufolge das gasförmige Nebenprodukt entfernt werden muß. Hierbei verursacht das entstehende Gas durch den gleichmäßigen Austrag keine Probleme durch Schäumen oder Totgasvolumen. In einer bevorzugten Ausgestaltung der Erfindung wird der Prozeß so geführt, daß im begasten Aufstiegsteil die zusätzlich entstehende Gasphase ausgegast und im Abstiegsteil durch Kompression teilweise in Lösung gehalten wird, wobei das gasförmige Nebenprodukt also noch aktiv zum Antrieb beiträgt. Im Schlaufenreaktor können auf diese Weise Reaktionen unter Gasaufnahme

- 4 -

0111885

und/oder -abgabe über lange Zeit ohne Aktivitätsverlust durchgeführt werden. Diese Verfahrensweise eignet sich insbesondere für kontinuierliche Reaktionen.

Die kontinuierliche Vergärung wäßriger Maischen ist bereits aus der DE-AS 2 938 339 bekannt. Bei diesem Verfahren wird neben Ethanol auch Hefe produziert; bezogen auf das eingesetzte Nährmedium ist also die Alkoholausbeute relativ gering. Demgegenüber verbraucht bei der erfindungsgemäß durchgeführten alkoholischen Vergärung der immobilisierte Biokatalysator praktisch kein Nährmedium, d.h. es findet kein Hefewachstum statt, weshalb der eingesetzte Zucker in signifikant höherer Ausbeute in Ethanol umgewandelt wird. Die erfindungsgemäße Reaktionsführung ist also nicht "wachstumsgekoppelt".

Enzymatische Reaktionen, bei denen eine pH-Verschiebung erfolgt, wie beispielsweise die fermentative Herstellung von Milchsäure, können optimal gesteuert werden. Es kommt somit zu keinem Aktivitätsabfall des Katalysators und damit zu gleichmäßigen hohen Ausbeuten. Die kontinuierliche Durchführung der Reaktion wird dadurch erheblich erleichtert.

In den folgenden Beispielen wird die Erfindung näher erläutert. Prozentangaben beziehen sich hierbei auf das Gewicht, wenn nichts Gegenteiliges angegeben ist. Die in den Beispielen 1 und 3 beschriebenen Immobilisierungsreaktionen sind Gegenstand der deutschen Patentanmeldung P 32 37 341.4.

Beispiel 1

600 g abzentrifugierte feuchte Hefe-Zellen (Saccharomyces cerevisiae) werden zusammen mit 1710 g Acrylamid und 90 g N,N'-Methylen-bis-acrylamid in 5 l physiologischer Kochsalzlösung aufgenommen und unter kräftigem Rühren zu einer Mischung von 20 l Perfluornonen-(1) und 20 ml eines nichtionischen Tensids, die zuvor durch Durchleiten von Stickstoff von Sauerstoff befreit wird, gegeben. Die Polymerisation wird bei 10°C mit 3 g Ammoniumpersulfat, gelöst in 20 ml 0,9 %iger Kochsalzlösung, und 5 ml N,N,N',N'-Tetramethylethylendiamin gestartet. Nach etwa 5 Minuten ist die Reaktion beendet. Die erhaltenen Biokatalysator-Perlen werden abfiltriert und mit physiologischer Kochsalzlösung gut gewaschen.

700 g dieser immobilisierten Hefe-Zellen werden mit 4 l 10%iger Glukosesirup-Lösung in einen Schlaufenreaktor mit einem Nutzvolumen von 7 l gegeben. Durch Anlegen eines geringen Stickstoffstroms an den Boden des Schlaufenreaktors wird der Reaktorinhalt derart in Bewegung gesetzt, daß im Innenrohr die Biokatalysatorperlen sich nach oben bewegen und im Außenrohr abfallen. Das bei der Reaktion entstehende Kohlendioxid entweicht am oberen Ende der Schlaufe. Nach Eintreten der Reaktion wird solange 70%iger Glukosesirup zugegeben, bis die Ethanolkonzentration 10 % erreicht. Anschließend wird 20%iger Glukosesirup zudosiert und gleichzeitig die gleiche Volumenmenge wäßriger Ethanol-Lösung über einen Seitenausgang im oberen Drittel der Reaktorschlaufe entnommen. Die Dosiermenge wird hierbei so eingestellt, daß die Ethanolkonzentration im Ablauf zwischen 9,5 und 10 % liegt. Der Umsatz beträgt hierbei 120 g Ethanol pro Stunde und pro l Biokatalysator. Nach 30 Tagen Reaktionszeit kann noch kein Aktivitätsabfall festgestellt werden.

Beispiel 2

600 g abzentrifugierte Hefe-Zellen (Saccharomyces cerevisiae) werden mit 600 g 5%iger Natriumalginatlösung versetzt und gerührt, bis eine homogene Mischung eintritt. Diese viskose Mischung wird durch eine Kanüle mit 1,0 mm Durchmesser in ein Vernetzerbad, bestehend aus 3 l einer 0,5 molaren Calciumchlorid-Lösung, getropft und 20 Minuten gerührt. Die erhaltenen Katalysatorkügelchen werden abfiltriert und wie in Beispiel 1 beschrieben eingesetzt.

0111885

Beispiel 3

300 g abzentrifugierte feuchte Lactobacillus bulgaricus-Zellen werden mit 855 g Acrylamid und 45 g N,N'-Methylen-bis-acrylamid in 3 l physiologischer Kochsalzlösung aufgenommen und unter kräftigem Rühren zu einer Mischung von 10 l Perfluornonan und 20 ml eines nichtionischen Tensids, die zuvor mit Stickstoff von Sauerstoff befreit wird, gegeben. Bei 10°C wird die Polymerisation mit 3 g Ammoniumpersulfat, gelöst in 20 ml 0,9 %iger Kochsalzlösung, und 5 ml N,N,N',N'-Tetramethylethylendiamin gestartet. Nach etwa 5 Minuten ist die Reaktion beendet. Die erhaltenen Perlen werden abfiltriert und mit physiologischer Kochsalzlösung gut gewaschen.

500 g der so immobilisierten Zellen werden mit 4 l einer 10%igen Glukoselösung in einen Schlaufenreaktor mit einem Nutzvolumen von 7 l gegeben. Durch Anlegen eines leichten Stickstoffstroms wird - wie in Beispiel 1 beschrieben - die Bewegungsrichtung vorgegeben. Zur Messung und Konstanthaltung des pH-Wertes des Reaktionsmediums sind an der Außenwand des Schlaufenreaktors 4 pH-Elektroden mit Einlaßdüsen angebracht.

Das Reaktionsgemisch wird auf 40°C erwärmt und diese Temperatur wird im gesamten Verlauf der Reaktion aufrechterhalten.

Der pH-Wert wird auf 6,0 eingestellt und ebenfalls durch Zugabe verdünnter Natronlauge konstant gehalten.

PATENTANSPRÜCHE:

1. Verfahren zur Durchführung enzymatischer Reaktionen mit immobilisierten Biokatalysatoren, dadurch gekennzeichnet, daß die Reaktion in einem Reaktor nach dem Schlaufen-prinzip erfolgt.

2. Verfahren nach Anspruch 1, dadurch gekennzeichnet, daß der Reaktor als Airliftreaktor betrieben wird.

3. Verfahren nach Anspruch 1 oder 2, dadurch gekennzeichnet, daß die Reaktion unter Gasaufnahme und/oder -abgabe verläuft.

4. Verfahren nach einem oder mehreren der vorhergehenden Ansprüche, dadurch gekennzeichnet, daß die Reaktion unter pH-Verschiebung verläuft.

5. Verfahren nach einem oder mehreren der vorhergehenden Ansprüche, dadurch gekennzeichnet, daß die Reaktion kontinuierlich betrieben wird.

Europäisches Patentamt

**EUROPÄISCHER RECHERCHENBERICHT**

**0111885**
Nummer der Anmeldung

EP 83 11 2559

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | CHEMICAL ABSTRACTS, Band 92, Nr. 17, April 1980, Seite 438, Nr. 144954m, Columbus, Ohio, US & JP - A - 79 117 379 (TOKYO RIKA KIKAI K.K.) 12-09-1979 * Abbildung * | 1-5 | C 12 M 1/08 C 12 P 7/06 C 12 M 1/40 |
| X | BIOTECHNOLOGY AND BIOENGINEERING, Nr. 12, Mai 1982, Seiten 147-159, New York, US A. MARGARITIS u.a.: "The use of immobilized cells of Zymomonas mobilis in a novel fluidized bioreactor to produce ethanol" * Page 147, "summary"; Abbildung 2 * | 1-5 | |
| E | EP-A-0 099 634 (UNIVERSITY OF WATERLOO) * Ansprüche; Abbildungen; Seite 2, Zeilen 23-34 * | 1-5 | RECHERCHIERTE SACHGEBIETE (Int. Cl. 3) C 12 P |

Der vorliegende Recherchenbericht wurde für alle Patentansprüche erstellt.

| Recherchenort | Abschlußdatum der Recherche | Prüfer |
|---|---|---|
| DEN HAAG | 21-03-1984 | COUCKE A.O.M. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03.82